# EUROPEAN PATENT SPECIFICATION

(11) **EP 1 951 267 B1**
(45) Date of publication and mention of the grant of the patent: **26.10.2011**
(21) Application number: 06828879.4
(22) Date of filing: 27.10.2006
(51) Int. Cl.: A61K 33/00, A61K 9/14, C01B 13/14

(54) **NANOPARTICLES OF A HETEROCRYSTAL MINERAL FOR USE AS A MEDICAMENT AND METHOD OF PRODUCING THE SAME**
NANOTEILCHEN EINES HETEROKRISTALL-MINERALS ZUR VERWENDUNG ALS MEDIKAMENT UND HERSTELLUNGSVERFAHREN DAFÜR
NANOPARTICULES DE MINÉRAUX HÉTÉROCRISTALLINS ET LEUR MÉTHODE DE FABRICATION

(30) Priority: 28.10.2005 EP 05077473
(43) Date of publication of application: 06.08.2008
(73) Proprietor: Kurkayev, Abdula, 1094 Budapest (HU)
(72) Inventor: Kurkayev, Abdula, 1094 Budapest (HU)
(74) Representative: Hatzmann, Martin
(86) International application number: PCT/EP2006/010383
(87) International publication number: WO 2007/048634

(56) References cited:
- EP-A- 0 684 208
- WO-A-00/02590
- WO-A-03/059834
- WO-A-2004/087765
- WO-A-2006/017337
- WO-A-2007/034586
- CN-A- 1 436 726
- US-A- 5 066 355
- US-A1- 2002 051 823
- US-A1- 2003 072 810
- DATABASE WPI Week 200572 Derwent Publications Ltd., London, GB; AN 2005-703321 XP002429929 & WO 2005/095621 A1 (GENOMIDEA INC) 13 October 2005 (2005-10-13) & WO 2005/095621 A (GENOMIDEA INC [JP]; NAKAGAMI HIRONORI [JP]; KANEDA YASUFUMI [JP]; NISH) 13 October 2005 (2005-10-13)

## Description

The invention relates to nanoparticles, in particular to nanoparticles for use in medicine.

An embodiment of a substance for a therapeutic use, based on its properties for generating chemically active reagents, is known from WO 96/26730. The known material comprises an organometallic compound, which is used for generating active chemical species at mildly acidic pH. The active chemical species act on cells causing their damage due to generation of active radicals and active oxygen in course of chemical reactions.

It is a disadvantage of the known substance that its chemical activity is highly dependent on the degree of acidity of the tissue, which has to be artificially kept at a certain level, by means of, for example, an application of hypo- or hyperthermia.

WO 03/059834 A1 relates to a composite material that comprises a glass or glass-ceramic phase, which glass or glass-ceramic phase has a surface that comprises nanoparticles. Amongst others, the use of the composite material for the preparation of a medical product is claimed.

EP-A 684 208 relates to ultrafine iron-containing rutile titanium dioxide particles, *e.g.* for use in medicinal compositions.

WO 2004/087765 A1 relates to a titanium dioxide complex capable of distinguishing molecules.

US 2003/0072810 relates to the use of antimicrobial metals to induce apoptosis and to inhibit matrix metalloproteinases in a disease condition.

WO 00/02590 relates to the use of nanparticles or microparticles in the treatment of a solid tumour.

US 2002/0051823 relates to a nanosilver-containing antibacterial and antifungal granule comprising nanoparticles each comprising a metallic silver core surrounded by silver oxide.

The abstract of WO 2005/095621 A1 describes the use of magnetic particles coated with a biomolecule for introducing said biomolecule to a target tissue.

WO 2006/017337 A1 relates to a composition for sustained drug delivery, comprising a drug and a ceramic structure. The ceramic structure comprises a metal oxide selected from a specified group of metal oxides.

WO 2007/034586 relates to photocatalytic titanium dioxide microparticles. The surface of the particles is modified with a cationic hydrophilic polymer.

None of these references disclose nanoparticles of a heterocrystal mineral selected from the group of SiO₂ heterocrystal minerals, quartzite, sphene, leukoxen and rutilated quartz for use as a medicament.

It is an object of the invention to provide a substance for use as a medicament which exhibits high intrinsic chemical and biologic activity.

To this end, according to the invention nanoparticles of a heterocrystal mineral as defined in claim 1 are provided for use as a medicament.

Within the terms of current invention, nanoparticles refer to dimensions of 0,5 - 200 nm, preferably to dimensions of 0, 5 - 100 nm, more preferably to dimensions of 0,5 - 50 nm. The nanoparticles may have spheric and/or rod shapes, their dimensions being measured by per se known Atomic Force Microscope (AFH) or Stonnich Tunnel (ST). It is noted that nanoparticles may comprise various mixtures of nanoparticle sizes, whereby also relative percentage of nanoparticles of specific dimensions may vary. The term dimension relates to all dimensions of nanoparticles. By the term heterocrystal mineral one understands a substantially chemically homogenous substance, developed in a crystal within different lattices with respect to their types and shapes yielding polymorphic material, which can be detected by a per se known DSC method.

Nanoparticles of the invention may in particular be use for one or more of the following: treatment of an infection, in particular a bacterial and/or viral infection; treatment of a cancer; wound healing; treating anemia; detoxification.

In the art it is understood that nanoparticles have dimensions comparable with dimensions of glucose molecule, and are thereby thousands of times smaller than an average size of a human cell. Therefore, nanoparticles can participate in chemical and biological reactions in a comparable way as molecules of matter, their properties being dependent on the properties of materials they are prepared from. An embodiment of a mineral (graphite) reduced to nanoparticles is known from RU 1263840. The known material is used as a filter for purifying water.

The technical measure of the invention is based on the insight that nanoparticles meet requirements for their application in medicine due to the fact that their high chemical activity is an intrinsic property and substantially no additional measures have to be taken to increase their specific chemical or biological activity in a recipient, it being a human or animal. The invention is based on a further insight that nanoparticles have a property of desorbing many earlier absorbed materials in aqueous medium, which is particularly useful for medical purposes. Nanoparticles of Si02 are of particular interest with his respect. Nanoparticles as such, notably comprising a bio-compatible composition for use as a medicament are contemplated as well.

Other minerals with a heterocrystal structure may be used to provide nanoparticles, notably rutile (Ti02; (Ti, Nb, Fe)02), sphene (CaO.SiO2,TiO2), loparite (Ti,Nb)2(Na, Ca, Ce)206), perowskite (CaTiO3), anatase (TiO2, (Ti, Nb, Fe)02), ilmenite (Fe1.10Ti90O3; FeTi03; Fe+2TiO3; (Fe, Mg)(Ti, Fe)O3, Fe+2TiO3, (Fe, Mg)TiO3; Fe+2TiO3/Fe+20.TiO2)), In addition to Si containing nanoparticles, e.g. leukoxen (TiO2Fe2O3+nH20/Cr, Al, Si, P, Nb, Zr, Tr, Ta) or quartzite (SiO2), ferrite (Fe2O3, FeO2). These minerals are abundant in nature and can easily be destructed to nanoparticle size. By the term heterocrystal mineral one understands a substantially chemically homogenous substance, developed in a crystal within different lattices with respect to their types and shapes yielding polymorphic material which can be detected by a per se known DSC method.

Preferred examples of heterocrystal minerals include leukoxen, sphene, and quartzite. In addition nanoparticles of a heterocrystal mineral selected from the group of Rutile loparite, perowskite, anatase, ilmenite, ferrite, barite, argyrite, graphite, calcium oxide, phosphoritemonooxides, phosphoritedioxides. and silver oxide, may further be present in a medicament of the invention.

Ferrites belong to a class of materials comprising dioxide of iron, most of them being ferromagnetic. Ferrites can refer to either a natural mineral or a chemically produced material using Fe2O3. Rutile is a natural mineral being a major ore of titanium, a metal used for high tech alloys because of its light weight, high strength and resistance to corrosion. Microscopic inclusions of rutile can be found in quartz, tourmaline, ruby, sapphire. Rutilated quartz can also be selected as a mineral source for production of nanoparticles. This stone is produced because at high temperatures and pressure, n(Si02)-n(TiO2) is in a stable state but as temperatures cool and pressure eases the two separate with rutile crystals trapped inside the quartz crystals. Other suitable heterocrystal minerals being sources of nanoparticles, notably comprising TiO2 comprise sphene, loparite, perowskite, anatase, ilmenite, leukoxen. Quartzite is a natural mineral composed almost entirely of quartz grains, derived from sandstone or from chert. Sandstones turn into quartzite in two different ways. The first way involves low pressure and temperature, where circulating fluids fill the spaces between sand grains with silica cement. When this rock is broken, the fractures go right through the original grains, not around them. This kind of quartzite, orthoquartzite, is not strictly speaking a metamorphic rock because the original mineral grains are still there, and bedding planes and other sedimentary structures are still evident. Under the high pressures and temperatures of deep burial, the mineral grains recrystallize and all traces of the original sediments are erased. The result is a true metamorphic rock, called metaquartzite.

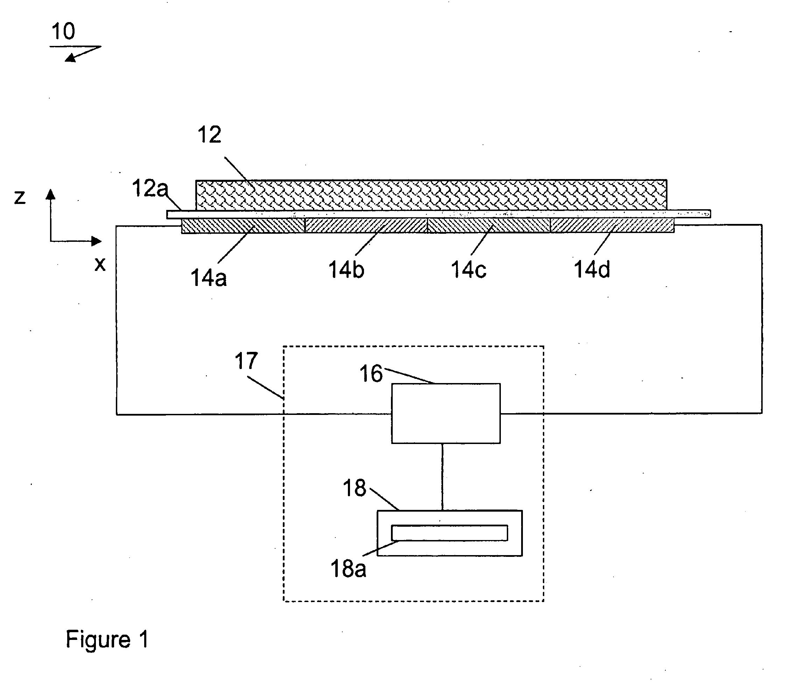

The technical measure of the invention is based on the insight that when a heterocrystal mineral is destructed to yield nanoparticles, the resulting nanoparticles comprise fragments of lattice with unsaturated bonds thereby providing particles with increased chemical activity, as active groups capable of initiating covalent and ionic bonds are notably formed at parts of intermolecular breaks. The technical measure of the invention is based on a further insight that practically all toxins, as well as inducers or mediators of pathologic processes in an organism belong to aggressive chemical compounds, having an elevated reaction potential for participating in chemical reactions of various kinds. Energetic neutralization of free chemical bonds of such toxic materials due to their saturation by means of complementary nanoparticles can stop predominantly all exogenic and edogenic intoxications. In case when the nanoparticles themselves doe not manifest any toxic effect, and can easily be excreted out of the organism, such class of materials is particularly advantageous for use as detoxifying agents. Therefore, nanoparticles can be used as a medicament as such. A wide class of minerals, for example, rutiles, ferrites, quartzites and so on belong to suitable materials for use as detoxifying medicaments, when reduced in size down to nanoparticles. These materials have heterocrystal structure, wherein lattice fragments are coupled by means of hydroxide ion (OH-). Preferably, these materials are subjected to a thermal destruction for purposes of production of nanoparticles, whereby a cooling mode is altered with a heating mode. In course of a cooling phase, the chemical bonds of the hydroxide ion undergo crystallization thereby destructing the lattice. The action of the alternating cooling/heating mode can be seen as internal blasts causing the matter to reduce its size down to nanometers, thereby yielding nanoparticles. Further details of the method of thermal destruction will be discussed with reference to Figure 1.

Next to their detoxifying action, as an additional effect, the nanoparticles of rutile, sphene, lopatire, perowskite, anatase, ilmenite, leukoxen, ferrite, quartzite exhibit anti-bacterial and anti-viral action and are suitable for use as a medicament with anti-viral or anti-bacterial action. They are applicable either for systemic administration, or for local application. Advantageously, the nanoparticles can be used for sanitary purposes, hygiene, including radiation protection and radiation hygiene. The anti-bacterial and anti-viral action of nanoparticles originating from rutile, sphene, lopatire, perowskite, anatase, ilmenite, leukoxen, ferrite and quartzite is based on their property to deactivate unsaturated chemical bonds of peptides, glycozydes and the like. Also, they can be used as coatings or applications on surgical threads, bandages. Alternatively, such nanoparticles can be used to coat intaersitial prothesis, like artificial cardiac valves, bypasses, etc. Therefore, such nanoparticles lend themselves for use as anti-bacterial and anti-viral material as such.

It is preferable to use minerals comprising dioxides of metals, like rutile, sphene, loparite, perowskite, anatase, ilmenite, lukoxen and ferrites for the source of nanoparticles, as the resulting nanoparticles can be used as a medicament to treat cancer due to their additional properties of a photosensitizer able to generate active oxygen, notably in singlet form when subjected to irradiation with light. The technical measure of the invention is based on the insight that nanoparticles of a dioxide of the biologically compatible metal have a plurality of advantageous effects when used as the photosensitizer. The nanoparticles have a maximum surface of contact, thereby improving the efficacy of the activation process, which is substantially a mass exchange process dependent on the area of reacting surfaces. Nanoparticles with dimensions of 0,5 - 200 nm are prepared, preferably with dimensions of 0,5 - 100 nm, still preferably with dimensions of 0,5 - 50 nm, because they are comparable in size with the molecule of glucose, still further increasing their chemical activity. The nanoparticles may have spheric and/or rod shapes, their dimensions being measured by per se known Atomic Force Microscope (AFH) or Stonnich Tunnel (ST). It is noted that the photosensitizer according to the invention may comprise various mixtures of nanoparticle sizes, whereby also relative percentage of nanoparticles of specific dimensions may vary. The term dimension relates to all dimensions of nanoparticles.

The nanoparticles of a metal-catalyst have a double action, first due to generic properties of nanoparticles, as indicated with reference to the foregoing, and, secondly, due to the properties of a photosensitizer, as they additionally catalyze a formation of active oxygen in response to irradiation with light, notably in a wide range of wavelengths, from ultra-violet to infrared range. Also, providing a photosensitizer in nanoparticle form has an additional advantageous effect of the matter being transparent to a wide range of wave lengths of light thereby improving an efficacy of the material response to light irradiation.

It is further noted that the photosensitizer comprising nanoparticles of titanium dioxide and iron dioxide, being a substantially transparent medium, can be activated by visible light. Therefore, such materials are perfectly suitable for use in a form of powder to be supplied locally on superficial targets. For example, a suitable amount of powder of nanoparticles comprising titanium-dioxide or iron dioxide can be applied to a target region and can be subjected to the ambient light, or, alternatively, it can be subjected to another activation, for example from a laser, beforehand or during a suitable clinical or cosmetic procedure. It is noted that for superficial application the active oxygen will be formed from the atmospheric oxygen and the action of the photosensitizer for catalyzing the production of active oxygen shall be as efficient. Active oxygen must be understood as oxygen with increased activation potential, notably (ε, Δ) singlet oxygen.

Alternatively, it is possible to prepare a suitably concentrated suspension from the nanoparticles prepared from rutile, sphene, loparite, perowskite, anatase, ilmenite, leukoxen or ferrite, which enables administration of the photosensitizer into a recipient systemically, intracavitary, or by means of injection. Preferred embodiments of a liquid suitable to be used in the invention comprise water, solution of a colloid, liquid comprising protein, for example albumin, or a physiologic salt solution. Preferably, the resulting suspension is prepared using a ratio of at least 0,5 mg of nanoparticles, notably titanium dioxide or iron dioxide per 1 liter of liquid. It is noted that minerals like rutile, sphene, loparite, perowskite, anatase, ilmenite, leukoxen and ferrite generally comprise a mass fraction of 10% - 30% of respective metal dioxides. Therefore, for those minerals the total mass of prepared nanoparticles should be selected at least 5 mg per 1 liter of liquid. It is further noted that when nanoparticles are being used as photosensitizer, they may act as catalysts for production of active oxygen forms, thereby not participating in chemical reactions. Therefore, the total amount of the photosensitizer is not a critical parameter for production of active oxygen, provided a minimum amount of 0,5 mg Ti02 or Fe02 or mixture thereof per liter of liquid is prepared. Because a surface interaction of the liquid with the ambient oxygen will not be sufficient to produce a necessary level of oxygenation of the suspension, oxygen can be supplied to the resulting suspension from a suitable vessel my means of a pump. Preferable partial pressure of oxygen within the suspension is about 40 - 100 mmHg, still preferably about 70 mm Hg. Preferably, the photosensitizer is activated by means of irradiation using a laser light with a wavelength in the range of 0,8 - 0,9 micrometer, which corresponds to a maximum absorption rate of oxygen and a region of optical transparency of tissues to light.

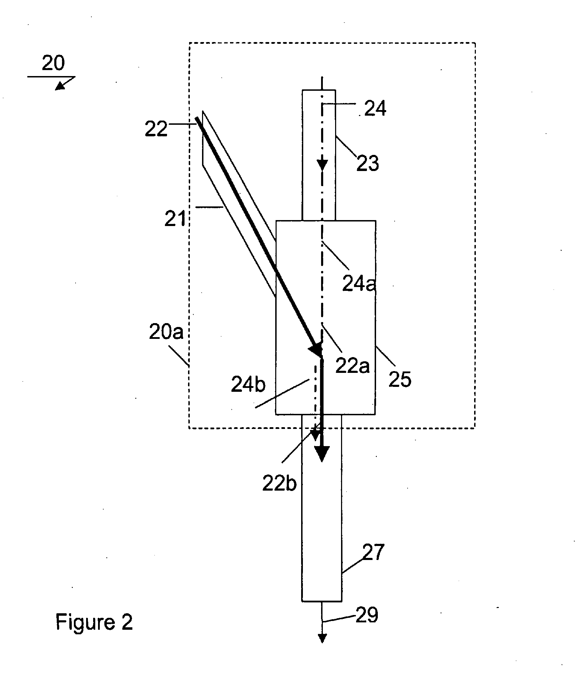

Still preferably, the photosensitizer is activated in a unit arranged to irradiate a suspension of the photosensitizer by a light source, notably a flow of the said suspension. By irradiating a flow of the suspension of the photosensitizer a production of active oxygen substantially in the whole volume of the suspension comprising photosensitizer is enabled. This step is advantageously carried out ex-vivo, for example as a preparatory step for photodynamic treatment of a suitable health disorder. Preferably, light intensity of at least 1 Joule/ml of the suspension comprising the photosensitizer is used. Still preferably, the light intensity is selected in the range of 2 -3 Joules/ml of the suspension of the photosensitizer. It is further preferable that the flow of the suspension comprising the photosensitizer is selected in the range of 1 - 2 ml/second. Preferably, a suitable Y-shaped body is used for a unit, whereby a first arm of the Y-shaped body is used to supply a flow of the suspension of the photosensitizer into a main channel of the unit and a second arm of the Y-shaped body is used to supply light into the main channel of the Y-shaped body, preferably, by means of a suitably selected and attached optical fiber. Preferably, the flow rate of the suspension of the photosensitizer is selected in accordance with the light intensity supplied by the optical fiber. In general, a preferable relation between the flow rate and the light intensity lies in the range of 2-5 W of light energy per 1 ml of the suspension. Still preferably, the main channel of the Y-shaped body is attached to a storage cavity for storing the activated photosensitizer prior to its use. Preferably, the cavity is prepared in a sterile manner, so that the required volume of the activated photosensitizer can be extracted by, for example, an injection needle in a course of a medical or cosmetic treatment. A preferred embodiment of the unit will be described in more detail with reference to Figure 2. Alternatively, the unit can be arranged to conduct the activated photosensitizer to a supply unit which is used for administering the activated photosensitizer into the target region. In this case the flow of the photosensitizer advantageously serves as an optic conductor for the light waves, thereby further focusing a deposition of the active oxygen in the target region.

Still preferably, the suspension is ozonated, for this procedure per se known apparata for ozonating liquids can be used. The advantage of this step is the provision of an increased concentration of oxygen in the medium comprising the photosensitizer and a production of a variety of active oxygen types (ε, Δ) in reaction to the activated photozensitizer. Preferably, the level of ozonation is kept within a range of 5 - 10 mg ozon per 1 liter of the suspension comprising the photosensitizer. The process of ozonation is advantageous, because it provides a variety of active oxygen forms in the suspension, like the singlet oxygen and ozone, which complement each other in their chemical and biological activity. Upon an interaction with other elements from environment the active oxygen and its secondary derivatives become a source of damage of a range of biologic objects which provide nutrition supply to cells. As a result, in the biological matter, subjected to an interaction with the compound according to the invention, fibrous processes are initiated, as well as processes of blood and lymphatic blockage, acting as mechanical barrier for blood and lymphatic circulation, thereby isolating, for example, a tumor from supply sources. As a result the metabolic activity and invasive properties of tumor cells are substantially reduced and the tumor cannot demonstrate its invasive and metastasic activity.

In a further alternative embodiment the mineral reduced to nanoparticles for use as a medicament comprises quartzite. Quarzites are characterized by presence of Si02, which in its nanoparticle form has an advantageous effect of initiating fibrous tissue. This advantageous effect, generally manifesting itself as a blood and lymphatic block, can be used for localization of cancerous tissue thereby for eliminating any source of nutrition of malignant cells. For administration of Si02 nanoparticles using injection a preferred dose of Si02 is about 10mg, for superficial applications there is no limitation. It is further noted that nanoparticles of Si02 can be used as transport agents, when chemically coupled to a suitable molecule. For example, nanoparticles of Si02 can be chemically coupled to silver oxides or dioxides, which can supplement the action of Si02 by its photosensitizing properties. Alternatively, a Si02-coupled Ag02 can be added to nanoparticles comprising dioxide of titanium or dioxide of iron thereby complementing their action as metal catalysts for production of active oxygen forms.

In a still further alternative embodiment, the nanoparticles of a heterocrystal mineral comprise a DNA-molecule are used as a medicament. Preferably, the nanoparticles are coupled to the DNA molecule, notably by electrostatic forces or by covalent bonds. It is noted that several embodiments of such coupling are envisaged, namely first, when a single nanoparticles is couples to a single DNA molecule, secondly, when a plurality of nanoparticles are coupled to a single DNA molecule, and third, when a plurality of DNA molecules are couples to a nanoparticle. This technical measure can be implemented by an introduction of, for example, sodium salt of a DNA-molecule, commercially known as a "Derinat", into a suspension comprising the said nanoparticles, preferably based on a proportion of 1 portion of 1,5% solution of sodium salt per 1 portion of nanoparticles comprising at least a dose of 0,5 mg of titanium dioxide, iron dioxide or silicon dioxide. When nanoparticles originating from Si02 are coupled to DNA molecule and, additionally to a suitable anti-metabolic agent, providing technical effect of improving deposition of the fibrogenic matter (SiO2) inside a cell in once case, and further damaging the cell by action of a anti-metabolic agent, in the other case. When both DNA molecule and anti-metabolic agent are coupled to nanoparticles, a synergistic effect may occur between the two effects set forth in the foregoing, still further improving a medical action of nanoparticles on the recipient. This embodiment has a technical effect of still further increasing a selectivity of bio-chemical action of the said nanoparticles due to the fact that they are transported inside the cell by action of the DNA-molecule.

Still preferably, the nanoparticles, notably, at least one of sphene, leukoxen, quartzite, comprise an anti-metabolic agent.

In particular such agents include anti-tumour agents with an anti-metabolic effect, cytostatic agents having an anti-metabolic effect and anticancer agents with an anti-metabolic effect. Anti-metabolic agents are generally known in the art.

This technical measure is based on the insight that the nanometric compound can be used as a transport agent to transport a further medicament inside a cell. The said coupling can be achieved by simply adding the anti-metabolic agent to the nanoparticles. A coupling of the anti-metabolic agent to the said nanoparticles provides a medicament with a cumulative cytotoxic action on the cell thereby still further improving an efficacy of the thus prepared substance. For example, the anti-metabolic agent may comprise a suitable chemotherapeutic material per se known in the art.

It is noted that although specific technical effects are explained with reference to isolated embodiments of nanoparticles produced by means of destruction of a heterocrystal mineral, various modifications of the embodiments, in particular, use of a mixture of disclosed minerals as a source of nanoparticles are contemplated as well. Nanoparticles for use as a medicament, notably nanoparticles comprising DNA molecule, and/or anti-metabolic agent are contemplated as well. It is further noted that production of the nanoparticles from the disclosed minerals provides a simple and economic way of effective production of medicaments as these minerals are present in nature in abundance. Some illustrations of the disclosed nanoparticles for use as medicaments will be discussed with reference to examples.

Example 1: nanoparticles from rutile or ferrite for use as a medicament for treating cancer.

This field of application is based on the photosensitizing property of dioxides of metals, it being a production of active oxygen forms under irradiation of light. For a superficially located cancerous mass the activated photosensitizer in nanoparticle form is introduced superficially and around the mass, for example, using a suitable injection needle. The volume of the suspension comprising the activated nanoparticle photosensitizer must be at least several times greater than the volume of the mass. Preferably, it is 5 to 10 times greater than the volume of the mass. It is noted that it is possible to provide the injection needle with a Y-shaped body one arm being a supply channel for the suspension comprising the photosensitizer, the other arm being an optical channel supplying the light. Preferably, the light intensity is not less than 1 W/cm2. Still preferably, the light is supplied in the direction of the flow of the suspension and is subjected to a further spreading in the tissue conceived to be treated. When laser is selected for the light source and is allowed to propagate inside a tissue being subjected to photodynamic treatment, it has an additional effect of inducing local hyperthermia still further improving medical effect of the procedure. The flow rate of the liquid is preferably not less than 1 ml per 1 Joules of light energy. Preferably, to reduce pain sensation of the recipient a suitable anesthetic is introduced into the suspension prior to the procedure of intervention. Preferably, a 0.2% lidocaine and 10mg/l ozon are used.

### Case 1. (reference)

A patient B. aged 18, (dossier No. 212/01) was treated in the year 2001 for a recidive of a soft-tissue sarcoma in a right half of the chest. The histologically proven recidive appeared after a time of three months of a tumor resection. During examination a spherical node of about 4 cm diameter was detected in a region of post-operative scar, said node being static, firmly attached to a periosteum of the front surface of II-III ribs. No distant metastases were detected by means of ultrasonic and X-ray investigations, however nodi lymphatici axillares were enlarged. The patient received interstitially 200 ml of the suspension comprising 1 mg of nanoparticles prepared by thermal destruction of rutile, irradiated by a laser light of 0,56 micrometer wavelength with an intensity of 5 W during 6 and 8 minutes. The procedure was repeated twice with a weekly interval. Tissue temperature in the course of treatment was not elevated by more than 1,4 degrees Celsius. A biopsy conducted three days after the second procedure demonstrated that the tumor belonged to sacroma's. After one year of follow-up no tumor growth was detected and in place of the recidive of 4cm in diameter one finds 1 cm node of fibrous tissue. During subsequent follow-up procedures no tumor manifestation was detected.

### Case 2. (referene)

For deep seated tumors which cannot be localized by a mere inspection, diagnostic means are used for presenting data on the spatial location of the target region comprising the tumor and the dimensions thereof. Also in this embodiment, a suitably arranged injection needle can be used for depositing the activated photosensitizer in the tumor and/or around it. A patient K, aged 64 (dossier No. 923) in November 2000 was hospitalized in an urologic clinic due to disurrhea. In the course of examination adenocarcinoma of an upper portion of the left lobe of prostate gland was detected (4 and 4 according to Glisson) Adjuvant to catheterization of the bladder, since 12.07.2001 a treatment by application of nanoparticles of rutile origin was carried out. Both lobes of the prostate gland received 250 ml of suspension comprising 1,5 mg dioxide of titanium in a solution of a silver dioxide irradiated by a laser light of 0.84 micrometer wavelength, intensity of 16 W/cm2, during 8 minutes. The tissue temperature was controlled and did not elevate by more than 1,2 degree Celsius in the course of treatment. The phenomenon of disurrhea has terminated and diagnostic screening showed solely a fibrous tissue in place of original tumor. This state was confirmed by a further follow-up three years after the treatment according to the invention. A biopsy (8 points) undertaken in Summer 2004 shoed no tumor cells and confirmed fibrosis of the gland tissues. January 2005 due to increased dysurics a transurethral resection performed. Some tissue fragments ablated within the surgical procedure detected "islands" of tumor surrounded by a firm healing tissue. No further evidences of a neoplastic process detected. April 2006, concentration of prostatic antigen is 2.1 mg/ml.

### Case 3.

A female patient L., age 59 (dossier No. 176/32) was included in a group of volunteer patients to receive treatment by means of administration of nanoparticles originating from the mineral according to the invention. The patient was diagnosed with a ductal type carcinoma of the left mamma, staged T3N2Mx. The radiotherapy received by the patient was not successful. During her investigation prior to treatment in accordance with the invention a tumor mass of substantial dimensions (10 cm3) was detected, which also had developed an ulcer of 30 cm2 area. In the course of treatment the patient received 10 times intro-paratumoural injections with activated photosensitizer comprising 20% titanium dioxide, 30 % of calcium dioxide, and at least 15% of silicon dioxide. 5 mg of the photosensitizer was used to produce a suspension of about 1 liter in volume using sterile water, whereto 5 ml of 1.5% Derinat was added and 5 mg of the anti-metabolic agent in the form of doxorubicin. Next to the intra- and para-tumoral injections the lymph nodes received the suspension of the activated photosensitizer as well. During a course of the follow-up no manifestation of cancerous cells in the treated volume was detected, which was confirmed by substantial hystologic analysis. The area of the ulcer defect was reduced by about 5 times and no signs of any cancerous process can be seen as well dermal defect of ulcer was closed by plastic surgery.

Example 2: use of nanoparticles prepared from heterostructure material for use as a medicament for wound healing.

### Case 4.

A female patient 76 years (case No. 318/A), after being a patient of a plurality of oncology clinics during a period of more than four years due to presence of a substantial (18 cm3) cancerous ulcer on her left check. Conventional attempts to close the dermal defect were not successful. Then she was subjected to a monthly course of treatment with nanoparticles prepared using a mixture of ferrite and quartzite, whereby the nanoparticles were administered in form of dry powder. Upon the application of the powder on the ulcer, it was irradiated with a halogen lamp with intensity of 3 W/cm2 in dose of 1,3 kJoule. After the treatment the skin defect disappeared, whereby the prints of ulcer showed forms of fibrous cells in phases of apoptosis. It is noted that although the present example shows a cumulative action of nanoparticles from ferrite and quartzite, the former was used to treat cancer and the latter was used as an adjuvant therapeutic agent for stimulating fibrosis to close the skin defect. It must be understood, that clinical use of nanoparticles prepared from quartzite for wound healing is contemplated hereby as such.

### Case 5.

A male patient 60 years after a composite operation for pancrease cancer manifested a small intestine fistula which could not be closed conventionally. One month later a layer of dry powder prepared from 60% Si02 and 70% Ti02 applied to the fistula area and presenting intestine during bandaging. After that within five minutes the area was irradiated with a Bioptrone device (20 mWt/dm2). In a week the purulent wound became quit and miniaturized. Within the second week the fistula closed by continuous bandaging.

Example 3: nanoparticles from heterostructure mineral (ferrite) as a medicament for treating anemia.

### Case 6. (reference)

A male patient, 31 years (case No. 1272/A) was operated in 2003 for melanoma of left half of his back, subjected to adjuvant chemotherapy and radiotherapy and therefore manifested acute anemia (hemoglobin level of 6,8 g/liter). He was treated with nanoparticles prepared from ferrite, the treatment was set forth with a background of non-healing wound at a place of surgical intervention. The treatment comprised 10 session of administration of 10mg of nanoparticles (22% mass fraction of iron dioxide) prepared in a total volume of 150 ml aqueous suspension. During injection of the thus prepared suspension, it was irradiated by a laser light with wavelength of 0,88 micrometer with a total dose of 1,4 kJoule. Already after the third injection the hemoglobin concentration in blood was increased by 50 % up to the level of 9 mg/l and the wound showed granulation process leading to total wound healing.

These and other aspects of the invention will be discussed in further details with reference to Figures.
Figure 1 shows in a schematic way an embodiment of an apparatus for production of nanoparticles using a thermal destruction method.
Figure 2 shows in a schematic way an embodiment of an Y-shaped body for activation of nanoparticles used as photosensitizer.

Figure 1 presents a schematic illustration of an embodiment of an apparatus 10 for production of nanoparticles using thermal destruction method. For this purpose a layer 12 of a heterocrystal mineral is provided on a suitable carrier 12a for enabling thermal contact with a suitable source 14a, 14b, 14c, 14d. Preferably, a thickness of the layer 12 in z-direction, schematically shown in Figure 1, is in the order of several millimeters, still preferably it is selected in the order of one millimeter, corresponding to a size of the lattice of rutiles, shpenes, loparites, anatases, ilmenites, leukoxenes, ferrites, quartzites. The dimension of the layer in x-direction, schematically shown in Figure 1, can be as large as one meter. Preferably, ceramics is selected for the carrier 12a, which can conduct energy in the range of 9 - 15 W/cm2. The sources 14a, 14b, 14c, 14d comprise thermo-elements, electrically coupled to a power supply source 16. Preferably, the power supply source is controlled by a processor 18, which is preferably operated by a suitable computer program 18a. Still preferably, the power supply unit 16 and the control unit 18 are implemented as an integral device 17. In accordance with the method of the invention, the power supply source produces pulses of constant current with amplitude of at least 10 A/mm2, which are transferred to the thermo-elements 14a, 14b, 14c, 14d. A thermo-element, conceived to operate in accordance with a per se known thermoelectric effect of Peltier-Zeebeck may be used in the method according to the invention, whereby upon application of a current of a suitable polarity the solder joints of the thermo-element act as coolers or as heaters. It is noted that application of a single thermo-element or a plurality of thermo-elements is contemplated. In an embodiment of the method according to the invention a pulse of a direct polarity is applied during, for example a period of 10⁻⁴ to 1 second, as a result of which temperature of the surface of thermo-element facing the layer 12 instantly decreases down to at least - 50 degrees Celcius, preferably to - 73 degrees Celcius. This causes the material of the layer 12 to substantially cool, whereby the hydroxide groups of water molecules crystallize thereby induce miniature blasts in the material of the layer 12. After this, the pulse of the direct polarity is changed to a pulse of an inverse polarity, thereby causing the surface of thermo-elements facing the layer 12 instantly to increase its temperature at least to + 80 degrees Celsius, preferably to +95 degrees Celsius, thereby melting water component in the crystal and disrupting lattice structure due to thermal vibration of lattice. After this, the polarity of the power supply unit 16 is altered again, causing the crystallization of water component. Preferably, such alteration is repeated at least for several times, for a period of, for example 1 sec to 1 minute. After a repetition of the cooling and heating modes, the layer 12 is destructed down to nanoparticles. A number of the repetitions can be selected in accordance with desired dimension of nanoparticles. In general, in accordance with the method of the invention nanoparticles with dimensions of 0,5-200 nm can be produced. It is also possible to produce a mixture of nanoparticles having various dimensions in the range of 0,5 - 200 nm. It is further possible to vary relative percentages of nanoparticles with specific dimensions in the mixture.

Figure 2 presents in a schematic way an embodiment 20 of a unit for activating a flow of the photosensitizer. The mixing unit 20a comprises a Y-shaped body, one arm 21 being conceived to receive, notably, a flow of the suspension of the photosensitizer 21, the other arm 23 being conceived to supply light waves 24 from a light source (not shown), preferably using a suitable optical fiber. The Y-shaped body of the unit 20a is arranged in such a way that the liquid photosensitizer 22a is fully irradiated by the light beam 24a. This arrangement ensures complete activation of the photosensitizer in the whole volume being irradiated. The portion 25 of the Y-shaped body may be manufactured with considerable dimensions, to contain the whole volume of the photosensitizer conceived to be used during a treatment procedure. It is found to be sufficient to provide the portion 25 with a 10 - 500 ml volume.

Preferably, the volume of the portion 25 is selected around 50 ml. When the photosensitizer is activated and is stored in the compartment 25, a supply unit 27 can be connected to its distal portion. The supply unit may comprise a catheter, an injection needle, or a spraying device used for administering the photosensitizer 29 to the target region of the recipient. In case when the injection needle, or a catheter are used it may be preferable not to interrupt the flow 22b of the activated photosensitizer allowing it to conduct the optical radiation into the tissues of the recipient. In this way the administration of the activated photosensitizer is performed substantially in real-time with its activation, whereby the liquid photosensitizer acts as an optical conductor of the light beam into the tissue. This procedure has an advantageous clinical effect as the nanometric photosensitizer instantly deposits the dose of the activated oxygen and the optical dose at the micro-level as well. In case when the laser light is used the optical dose thus delivered causes additional tissue damage due to local ablation due to laser-induced hyperthermia. Preferably, the laser light with the wavelength of 0,8 - 0,9 micrometer is used, corresponding to the maximum absorption rate of oxygen and the region of optical transparency of tissue.

## Claims

1. Nanoparticles of a heterocrystal mineral selected from the group of SiO₂ heterocrystal minerals, quartzite, sphene, leukoxen and rutilated quartz for use as a medicament, the nanoparticles having dimensions of 0.5-200_nm.

2. Nanoparticles for use as a medicament according to Claim 1, wherein the nanoparticles are selected from the group of sphene and rutilated quartz.

3. Nanoparticles for use as a medicament according to Claim 1 or 2, wherein the medicament further comprises nanoparticles of at least one of mineral selected from the group of loparite, perowskite, ilmenite, ferrite, anatase, rutile, barite, argyrite, graphite, calcium oxide, phosphoritemonooxides, phosphoritedioxides and silver oxide.

4. Nanoparticles for use as a medicament according to any one of the preceding Claims 1, 2 or 3, wherein nanoparticles comprise a DNA molecule.

5. Nanoparticles for use as a medicament according to Claims 1-4, wherein the nanoparticles comprise an anti-metabolic anti-tumour agent.

6. A composition comprising nanoparticles as defined in any one of the preceding Claims 1-5 and a fluid, preferably water or air, for use as a medicament.

7. Nanoparticles of a heterocrystal mineral for use as a medicament according to any one of the preceding Claims 1-4 for treatment of an infection, preferably a bacterial or a viral infection.

8. Nanoparticles of a heterocrystal mineral for use as a medicament according to any one of the preceding Claims 1 - 5 for treatment of cancer.

9. Nanoparticles of a heterocrystal mineral for use as a medicament according to Claims 1 - 5 for healing a wound.

## Patentansprüche

1. Nanoteilchen eines Heterokristallminerals, das aus der Gruppe ausgewählt ist, die aus SiO₂-Heterokristallminerallen, Quarzit, Sphen, Leukoxen und rutiliertem Quarz besteht, zur Verwendung als Medikament, wobei die Nanoteilchen Abmessungen von 0,5 bis 200 nm aufweisen.

2. Nanoteilchen zur Verwendung als Medikament gemäß Anspruch 1, wobei die Nanoteilchen aus der Gruppe ausgewählt sind, die aus Sphen und rutiliertem Quarz besteht.

3. Nanoteilchen zur Verwendung als Medikament gemäß Anspruch 1 oder 2, wobei das Medikament weiterhin Nanoteilchen wenigstens eines Minerals umfasst, das aus der Gruppe ausgewählt ist, die aus Loparit, Perowskit, Ilmenit, Ferrit, Anatas, Rutil, Baryt, Argyrit, Graphit, Calciumoxid, Phosphoritmonoxiden, Phosphoritdioxiden und Silberoxid besteht.

4. Nanoteilchen zur Verwendung als Medikament gemäß einem der vorstehenden Ansprüche 1, 2 oder 3, wobei die Nanoteilchen ein DNA-Molekül umfassen.

5. Nanoteilchen zur Verwendung als Medikament gemäß den Ansprüchen 1 bis 4, wobei die Nanoteilchen ein antimetabolisches Antitumormittel umfassen.

6. Zusammensetzung, die Nanoteilchen gemäß einem der vorstehenden Ansprüche 1 bis 5 und ein Fluid, vorzugsweise Wasser oder Luft, umfasst, zur Verwendung als Medikament.

7. Nanoteilchen eines Heterokristallminerals zur Verwendung als Medikament gemäß einem der vorstehenden Ansprüche 1 bis 4 zur Behandlung einer Infektion, vorzugsweise einer bakteriellen oder viralen Infektion.

8. Nanoteilchen eines Heterokristallminerals zur Verwendung als Medikament gemäß einem der vorstehenden Ansprüche 1 bis 5 zur Behandlung von Krebs.

9. Nanoteilchen eines Heterokristallminerals zur Verwendung als Medikament gemäß den Ansprüchen 1 bis 5 zur Heilung einer Wunde.

## Revendications

1. Nanoparticules d'un minéral hétérocristallin choisi dans le groupe comprenant les minéraux hétérocristallins à base de SiO₂, la quartzite, le sphène, le leucoxène et le quartz rutilé, pour une utilisation en tant que médicament, les nanoparticules ayant des dimensions de 0,5 à 200 nm.

2. Nanoparticules pour une utilisation en tant que médicament selon la revendication 1, lesquelles nanoparticules sont choisies dans le groupe comprenant le sphène et le quartz rutilé.

3. Nanoparticules pour une utilisation en tant que médicament selon la revendication 1 ou 2, dans lesquelles le médicament comprend en outre des nanoparticules d'au moins un minéral choisi dans le groupe comprenant la loparite, la perovskite, l'ilménite, la ferrite, l'anatase, le rutile, la baryte, l'argyrite, le graphite, l'oxyde de calcium, les monoxydes phosphorites, les dioxydes phosphorites et l'oxyde d'argent.

4. Nanoparticules pour une utilisation en tant que médicament selon l'une quelconque des revendications 1, 2 et 3 précédentes, lesquelles nanoparticules comprennent une molécule d'ADN.

5. Nanoparticules pour une utilisation en tant que médicament selon l'une quelconque des revendications 1 à 4, lesquelles nanoparticules comprennent un agent antitumoral antimétabolique.

6. Composition comprenant des nanoparticules telles que définies dans l'une quelconque des revendications 1 à 5 précédentes et un fluide, de préférence de l'eau ou de l'air, pour une utilisation en tant que médicament.

7. Nanoparticules d'un minéral hétérocristallin pour une utilisation en tant que médicament selon l'une quelconque des revendications 1 à 4 précédentes, pour le traitement d'une infection, de préférence d'une infection bactérienne ou virale.

8. Nanoparticules d'un minéral hétérocristallin pour une utilisation en tant que médicament selon l'une quelconque des revendications 1 à 5 précédentes, pour le traitement d'un cancer.

9. Nanoparticules d'un minéral hétérocristallin pour une utilisation en tant que médicament selon l'une quelconque des revendications 1 à 5 précédentes, pour la cicatrisation d'une plaie.
